# EUROPEAN PATENT APPLICATION

(11) **EP 0 668 084 A1**
(43) Date of publication of application: **23.08.1995**
(21) Application number: 94308175.2
(22) Date of filing: 07.11.1994
(51) Int. Cl.: A61M 1/00

(54) **Method and arrangement in connection with a secretion receiver**

(30) Priority: 16.02.1994 FI 940721
(71) Applicant: Muoviserres Oy, SF-61840 Kauhajoki (FI)
(72) Inventor: Rajamäki, Veikko, FI-61800 Kauhajoki (FI)
(74) Representative: Lord, Hilton David

(57) **Abstract**

The invention relates to a method and an arrangement in connection with a secretion receiver. In the invention, secretion is sucked by a vacuum source (6) into a secretion receptacle (2) through a patient hose (4) fitted in a first connection means (3) provided in a cover portion (1) of the secretion receptacle (2), and solidifying and disinfecting agent (8) is mixed with the secretion so as to solidify it. To facilitate the dispensing of the solidifying and disinfecting agent, the solidifying and disinfecting agent (8) is positioned in the first connection means by means of a container (7) in such a way that it is dispensed automatically under the influence of vacuum created by the vacuum source (6) without any separate measures.

## Description

The invention relates to a method in connection with a secretion receiver, wherein secretion is sucked by a vacuum created by a vacuum source into a secretion receptacle through a patient hose fitted in a first connection means of the secretion receptacle, and wherein solidifying and disinfecting agent is mixed with the secretion so as to solidify it. The invention also relates to an arrangement in connection with a secretion receiver, comprising a secretion receptacle, a first connection means for connecting a patient hose in flow connection with the secretion receptacle, and a second connection means for connecting a vacuum source to the secretion receptacle, the arrangement further comprising a container and containing solidifying and disinfecting agent for solidifying the secretion.

The invention concerns a secretion receiver system for medical uses, for instance. The invention is intended for daily use e.g. in the hospital in connection with secretion receivers.

The use of secretion receivers in the hospital has a number of risks, such as the risk of infection. Problems occur with such receivers particularly when the receiver leaks for some reason or when the receiver is broken so that the secretion escapes from the receiver. In certain cases, the required cleaning measures may be very difficult to carry out. The secretion receiver is also rather difficult to handle, as liquid secretion may spill and splash very easily. Spilled secretion may also cause problems if it enters the suction device.

To avoid the over-described problems, systems have been developed recently which use a special solidifying and disinfecting agent which solidifies and disinfects the secretion, so that the secretion will not splash and, in cases of accident, cleaning is considerably easier to carry out than previously. The solidifying and disinfecting agent is dispensed in a separate step with a suitable dispenser into the secretion.

The main problem with the prior art technique has been the separate dispensing step of the solidifying and disinfecting agent, as risk situations may thus occur when the agent is handled and dispensed. Another problem is that the dispensing of the solidifying and disinfecting agent may sometimes be forgotten. If the agent is dispensed afterwards, the solidification of the secretion by the agent is retarded, in addition to which the tasks of the hospital personnel will be hampered and retarded particularly when the secretion receiver is to be changed during a medical procedure.

The object of the invention is, in fact, to provide an arrangement, by means of which the disadvantages of the prior art can be eliminated. This is achieved by a method and an arrangement according to the invention. The method according to the invention is characterized in that the solidifying and disinfecting agent is positioned in the first connection means in such a way that it will be dispensed automatically under the influence of vacuum created by the vacuum source without any separate measures. The arrangement according to the invention in turn is characterized in that the container is positioned in the first connection means in such a way that a vacuum created by the vacuum source is arranged to cause the solidifying agent to be dispensed automatically without any separate measures.

A major advantage of the invention is that it allows the level of hospital hygiene to be improved substantially over the prior art technique, as the solidification eliminates efficiently the problems caused by leaking and spreading of secretion. In addition, the invention prevents drops of secretion formed by splashing from entering into the vacuum source creating a suction effect in the secretion receiver. Another advantage of the invention is that it is simple, whereby it is advantageous to introduce and use. Preferably, the first connection means is provided in a cover portion, the container being positioned in the cover portion. The cover portion may be arranged to close the secretion receptacle. The second connection means may be positioned in the cover portion.

In the following the invention will be described more fully by means of the embodiments shown in the attached drawings, whereby
Figure 1 is a general side view of a first embodiment of the arrangement according to the invention;
Figure 2 is a general side view of a second embodiment of the arrangement according to the invention;
Figure 3 is a general side view of a third embodiment of the arrangement according to the invention;
Figure 4 is a general side view of a fourth embodiment of the arrangement according to the invention;
Figure 5 is a general side view of a fifth embodiment of the arrangement according to the invention;
Figure 6 is a general side view of a sixth embodiment of the arrangement according to the invention.

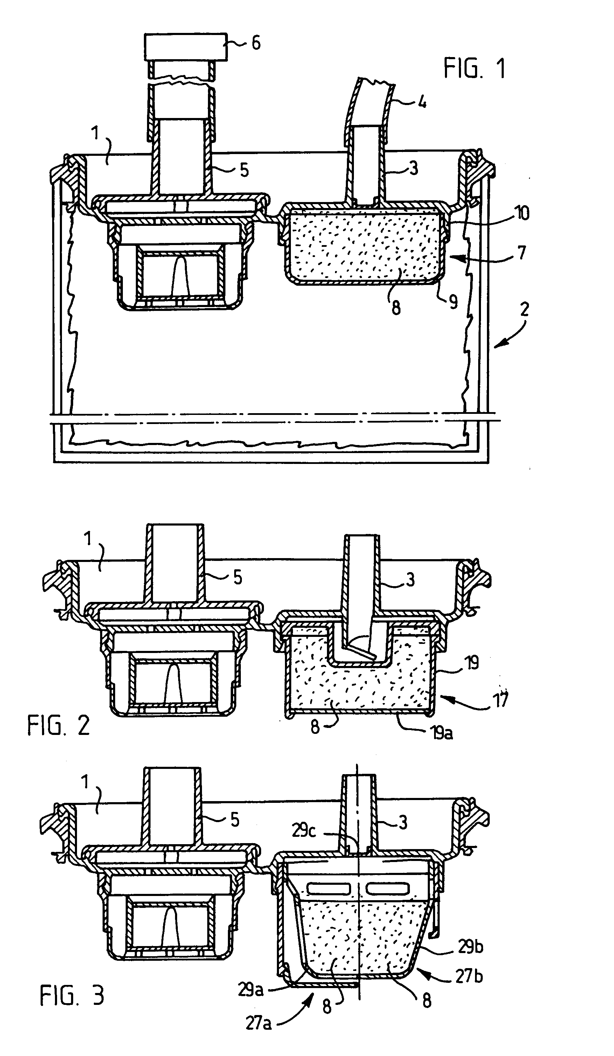

Figure 1 is a general view of one embodiment of the arrangement according to the invention. The reference numeral 1 indicates a cover portion, and the reference numeral 2 an actual secretion receptacle. The secretion receptacle may comprise a disposable bag or a reusable receptacle or their combination. Figure 1 shows a solution, where a disposable bag is positioned inside a reusable receptacle. The cover portion 1 is arranged to be positioned tightly upon the actual secretion receptacle 2 so as to close it. The cover portion 1 is provided with a first connection means 3 which is arranged to connect a patient hose 4 into flow connection with the secretion receptacle 2. The cover portion is also provided with a second connection means 5 for connecting a vacuum source 6 to the secretion receptacle.

The structure and operation of the secretion receptacle, hoses and vacuum source are fully obvious to one skilled in the art, so they will not be described more fully herein. It is only to be noted in this connection that the patient hose 4 is connected to the patient, and a vacuum created by the vacuum source 6 in the secretion receptacle 2 sucks secretion from the patient through the patient hose 4 into the secretion receptacle 2.

According to an essential basic idea of the invention, a container 7 with solidifying and disinfecting agent 8 is provided in connection with the first connection means 3 in the cover portion 1. The solidifying and disinfecting agent may be any suitable agent commercially available. One example would be a substance sold under the name Sanisorb and manufactured by Premium Plastics Inc., USA. The container 7 may be installed in position either at the factory stage or afterwards. To solidify the secretion, vacuum created by the vacuum source 6 is arranged to provide automatically, without any separate measures, a contact between the solidifying and disinfecting agent 8 and the secretion flowing through the patient hose 3. The idea of the embodiment shown in Figure 1 is that the solidifying and disinfecting agent is arranged in connection with the secretion receptacle 2 in such a way that a vacuum connected to the secretion receptacle 2 from the vacuum source 6 detaches the container 7, thus allowing the solidifying and disinfecting agent 8 to fall into the secretion receptacle 2, and the vacuum is admitted into the patient hose 4, by means of which secretion is sucked into the secretion receptacle 2. In this embodiment, an appropriate amount of the solidifying and disinfecting agent will always be positioned in the secretion receptacle when secretion flows into it.

In the embodiment shown in Figure 1, the container 7 is formed by a cup-like bottom portion 9 attached to a retainer 10 positioned inside the cover portion 1. The cup-like bottom portion may also be made of a number of parts. As used herein, the term inside means that surface of the cover portion 1 which will be positioned within the secretion receptacle when the cover portion is mounted upon the secretion receptacle 2. The idea is that the bottom portion 9 is detached under the influence of vacuum and falls into the secretion receptacle 2 so that the vacuum is able to act on the patient hose and thus suck secretion from the patient into the secretion receptacle, where it comes into contact with the solidifying and disinfecting agent that has dropped into it. An appropriate amount of the solidifying and disinfecting agent will be dispensed in advance into the receptacle portion; container portions may, for instance, be supplied with solidifying and disinfecting agent provided within them.

It is to be noted that the solidifying and disinfecting agent 8 will be mixed with the secretion fully automatically when the vacuum source 6 is switched on. In this embodiment, all of the solidifying and disinfecting agent 8 is arranged to be mixed with the secretion at the same time. The solidifying and disinfecting agent 8 may be e.g. in powder form.

Figure 2 shows a second embodiment of the arrangement according to the invention. In Figure 2, only the cover portion 1 is shown for the sake of clarity. In other respects, the structure is similar to the embodiment shown in Figure 1. This embodiment corresponds essentially to that shown in Figure 1, the only difference being the structure of a container 17. In this embodiment, the container 17 is formed by a bottom portion 19a and a wall portion 19b. The wall portion 19b is attached to a retainer 20 provided in the cover portion 1, and the bottom portion correspondingly to the lower edge of the wall portion. The idea of this embodiment is that the bottom portion 19a is detached by the influence of vacuum, so that the solidifying and disinfecting agent 8 will make contact with secretion flowing through a patient hose and flow openings formed in the wall portion, whereby the solidifying and disinfecting agent solidifies and disinfects the secretion. It is also possible that the wall portion 19b is detached and then falls into the secretion receptacle. The operation is similar to that described with reference to Figure 1.

Figure 3 shows two versions of a third embodiment of the invention. In this embodiment, a vacuum created in the secretion receptacle detaches a closing part 29c fitted in the first connection means 3, whereby secretion from the patient is able to flow into the receptacle portion 27a or 27b. In this embodiment, the wall portion 29a or 29b of the container remains attached to the cover portion. The idea is that the apparatus in a way dispenses solidifying and disinfecting agent 8 into the secretion while suction is effected. The flow takes place through openings formed in the wall portions 29a and 29b. In this embodiment, the solidifying and disinfecting agent 8 can be in the container e.g. in powder form.

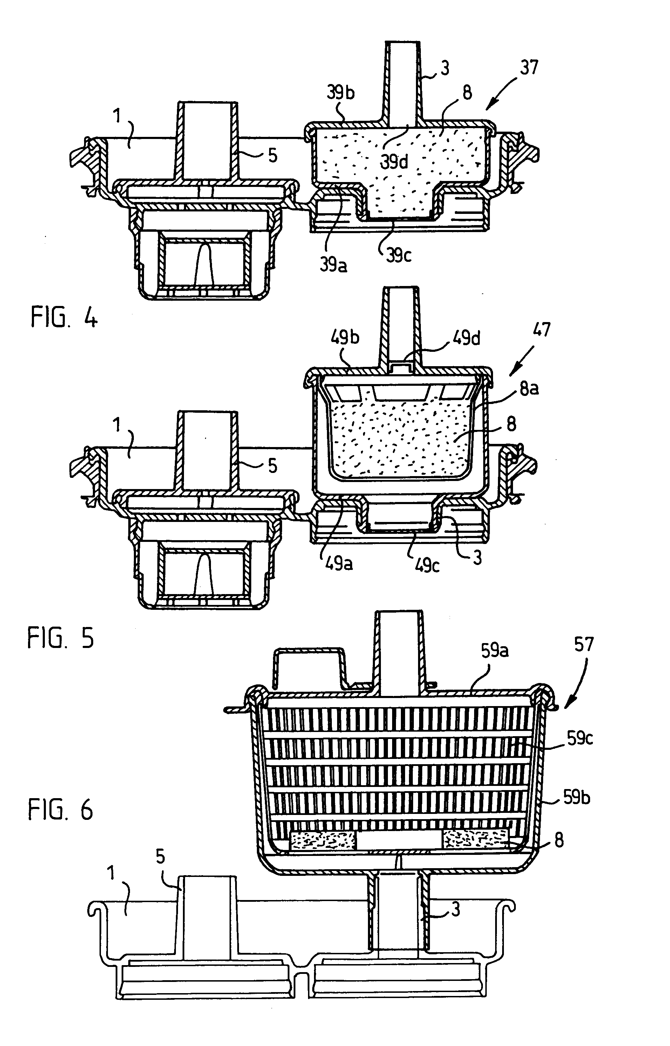

Figure 4 shows a fourth embodiment of the arrangement according to the invention. In this embodiment the container 37 is positioned outside the cover portion 1. The container 37 comprises a cover 39a, a wall portion 39b, and closing parts 39c and 39d. The idea is that the closing parts 39c and 39d open under the influence of vacuum, whereby the solidifying and disinfecting agent 8 falls into the secretion receptacle. In principle, the operation is similar to that described with reference to the example of Figure 1.

Figure 5 shows a fifth embodiment of the arrangement according to the invention. In this embodiment, the container 47 is, in principle, similar to that of the example shown in Figure 4, i.e. it comprises a cover 49a, wall portions 49b, and closing parts 49c and 49d. The closing parts are opened as described with reference to Figure 4. In this embodiment, it is essential that all of the solidifying and disinfecting agent 8 is not released at the same time, but it is dissolved or entrained in secretion flowing through the container 47 into the secretion receptacle, where the solidification and disinfection take place as described above. A characteristic feature of this embodiment is that the solidifying and disinfecting agent is packed in a separate cup or the like 8a, which is fitted in the container 47. In this embodiment, it is preferable that the solidifying and disinfecting agent is in solid form, as it may be difficult in certain conditions to hold a powder-like material in the cup 8a.

Figure 6 shows a sixth embodiment of the arrangement according to the invention. In this embodiment, the container 57 comprises a cover 59a, a wall portion 57b, and a screen portion 57c. In this embodiment, the solidifying and disinfecting agent 8 is in the form of a ring-shaped tablet arranged on the bottom of the screen portion 59c. Secretion removed from the patient under the influence of vacuum comes into contact with the solidifying and disinfecting agent, which is dissolved in the secretion and entrained with it. In this embodiment, secretion flows through the container 57, so that the solidifying and disinfecting agent is gradually dissolved in the flow of secretion. In other respects, the operation corresponds to the operating principles disclosed in connection with the embodiments disclosed above. This embodiment advantageously allows solidifying and disinfecting agent to be used only when it is needed, as the container can be attached rapidly to the first connection means, if required. The arrangement shown in Figure 6 is suitable for existing secretion receiving bags. The version shown in Figure 6 can be advantageously supplied to hospitals as a separate part.

The embodiments described above are by no means intended to restrict the invention, but the invention can be modified at will within the scope of the claims. Accordingly, it is obvious that the arrangement according to the invention or its details need not necessarily be exactly similar to those shown in the figures, but other solutions are possible as well.

## Claims

1. Method in connection with a secretion receiver, wherein secretion is sucked by a vacuum created by a vacuum source (6) into a secretion receptacle (2) through a patient hose (4) fitted in a first connection means (3) of the secretion receptacle (2), and wherein solidifying and disinfecting agent (8) is mixed with the secretion so as to solidify it, characterized in that the solidifying and disinfecting agent (8) is positioned in the first connection means in such a way that it will be dispensed automatically under the influence of vacuum created by the vacuum source (6) without any separate measures.

2. Arrangement in connection with a secretion receiver, comprising a secretion receptacle (2), a first connection means (3) for connecting a patient hose (4) in flow connection with the secretion receptacle, and a second connection means (5) for connecting a vacuum source (6) to the secretion receptacle, the arrangement further comprising a container containing solidifying and disinfecting agent (8) for solidifying the secretion, characterized in that the container (7, 17, 27a, 27b, 37, 47, 57) is positioned in the first connection means (3) in such a way that a vacuum created by the vacuum source (6) is arranged to cause the solidifying agent (8) to be dispensed automatically without any separate measures.

3. Arrangement according to Claim 2, characterized in that the secretion receiver comprises a cover portion, the cover portion being provided with the first connection means and the container being provided in the cover portion.

4. Arrangement according to Claim 3, characterized in that the container (7, 17, 27a, 27b) is positioned inside the cover portion (1).

5. Arrangement according to Claim 3, characterized in that the container (37, 47, 57) is positioned outside the cover portion (1).

6. Arrangement according to any of the preceding Claims 2 to 5, characterized in that the container (7, 17, 37) is arranged to open under the influence of vacuum created by the vacuum source (6).

7. Arrangement according to Claim 6, characterized in that all of the solidifying and disinfecting agent (8) is arranged to drop at the same time into the secretion receptacle before the secretion flows into the receptacle.

8. Arrangement according to any of the preceding Claims 2 to 5, characterized in that the solidifying and disinfecting agent (8) is arranged to gradually dissolve in the flow of secretion as the secretion flows through the container (27a, 27b, 47, 57).

9. Arrangement according to any of the preceding Claims 2 to 7, characterized in that the solidifying and disinfecting agent (8) is in powder form.

10. Arrangement according to Claim 8, characterized in that the solidifying and disinfecting agent (8) is a solid body soluble in secretion.

11. Apparatus for use with a secretion receiver of the type comprising a secretion receptacle, a first connection means (3) for connecting a patient hose (4) in flow connection with the secretion receptacle, and a second connection means (5) for connecting a vacuum source (6) to the secretion receptacle, the apparatus comprising a container for containing solidifying and disinfecting agent (8) for solidifying the secretion, characterized in that the container (7, 17, 27a, 27b, 37, 47, 57) is positionable in the first connection means (3) in such a way that a vacuum created by the vacuum source (6) is arranged to cause the solidifying agent (8) to be dispensed automatically without any separate measures.
